# EUROPÄISCHE PATENTSCHRIFT

(11) **EP 0 761 765 B1**
(45) Veröffentlichungstag und Bekanntmachung des Hinweises auf die Patenterteilung: **18.10.2000**
(21) Anmeldenummer: 96113568.8
(22) Anmeldetag: 23.08.1996
(51) Int. Cl.: C09B 61/00, A23L 1/275, C07C 403/00

(54) **Verfahren zur Extraktion von Carotinfarbstoffen aus Naturstoffen**
Process for the extraction of carotene dyes from natural products
Procédé pour l'extraction de colorants de carotène à partir de produits naturels

(30) Priorität: 25.08.1995 DE 19531254
(43) Veröffentlichungstag der Anmeldung: 12.03.1997
(73) Patentinhaber: SKW Trostberg Aktiengesellschaft, 83308 Trostberg (DE)
(72) Erfinder: Heidlas, Jürgen, Dr., 83308 Trostberg (DE); Huber, Georg, 83352 Altenmarkt (DE); Cully, Jan, Dr., 84518 Garching (DE); Kohlrausch, Utz, Dr., 83308 Trostberg (DE)
(74) Vertreter: Weiss, Wolfgang, Dipl.-Chem. Dr.

(56) Entgegenhaltungen:
- EP-A- 0 278 284
- EP-A- 0 455 425
- DE-A- 4 342 798
- DE-A- 4 429 506
- US-A- 5 310 554
- US-A- 5 378 369

## Beschreibung

Die vorliegende Erfindung betrifft ein Verfahren zur Extraktion von Carotinfarbstoffen, das es mit Hilfe der Lösemitteltechnologie, durch die Wahl geeigneter Lösemittelgemische sowie entsprechender Verfahrenstemperaturen ermöglicht, aus festen, trockenen oder getrockneten Naturstoffen Carotinfarbstoffe, insbesondere β-Carotin, zu extrahieren.

Carotinoidfarbstoffe sind in der Natur weitverbreitet und verleihen vielen Naturstoffen, wie z.B. Karotten, Paprikaschoten, Blüten (Tagetes) oder bestimmten Mikroorganismen, ihre charakteristische gelbe bis tiefrote Färbung. Ausgehend von deren chemisch-strukturellen Unterschieden wird die Klasse der Carotinoide in zwei Unterklassen aufgeteilt: Carotine, die chemisch gesehen reine Kohlenwasserstoffe sind, und Xantophylle, die oxygenierte Funktionen (-OH, -O-, =O) im Molekül enthalten. Aufgrund ihrer Unterschiede in der chemischen Struktur zeigen Carotine und Xantophylle, die beide als natürliche Fettbegleitstoffe vorkommen, ein unterschiedliches Löseverhalten in organischen Lösemitteln.

Carotinverbindungen allgemein, insbesondere aber das β-Carotin, erlangten in der jüngsten Vergangenheit eine stetig zunehmende wirtschaftliche Bedeutung.

Zum einen wird β-Carotin seit langem in der Lebensmitteltechnologie als färbender Zusatzstoff (Farbstoff) oder als Antioxidans eingesetzt, wobei das Anwendungsgebiet nicht auf lipide Systeme beschränkt ist, sondern durch den Einsatz besonderer Technologien, wie z.B. diverse Einkapselungen des β-Carotins auch auf wäßrige Systeme ausgedehnt worden ist. Andererseits hat das β-Carotin in der Diätetik eine besondere Bedeutung, da ihm physiologisch die Funktion als Provitamin A zukommt. Insbesondere durch neuere wissenschaftliche Erkenntnisse, daß β-Carotin auch systemisch als Antioxidans und Radikalfänger wirken und darüberhinaus nachweislich Beiträge in der Krebsprophylaxe liefern kann (Erdmann, J.W., Grummer, M.A., Does β-Caroteen Consumption lower Cancer Risk? Backgrounder vol.1, no.1, Vitamin Nutrition Service, USA, 1984), führten dazu, daß es vermehrt in das Interesse einer größeren Verbrauchergruppe gerückt ist.

Der damit gestiegenen Nachfrage versucht die Industrie einerseits durch die Herstellung von synthetischem β-Carotin und andererseits durch die Extraktion und anschließende Kristallisation von β-Carotin aus natürlichen Quellen zu entsprechen. Dabei geben die Verbraucher entsprechend ihrem gegenwärtig kritischen Bewußtsein gegenüber synthetischen Produkten dem natürlichen β-Carotin eindeutig den Vorzug.

Während noch vor relativ kurzer Zeit ausschließlich die "klassischen" natürlichen β-Carotinquellen, wie z.B. Karotten oder Algen, für wirtschaftliche Anreicherungsverfahren zur Verfügung standen, ist es in jüngster Zeit durch innovative Anstrengungen der Biotechnologie gelungen, mit fermentativen Methoden eine ungleich besser geeignete, natürliche Quelle zu erschließen. Durch die Fermentation bestimmter sproßbildender Pilze kann heute eine Konzentration von bis zu über 5 Gew.-% β-Carotin in der getrockneten Fermentationsbiomasse erreicht werden; die Konzentration an β-Carotin liegt damit um Zehnerpotenzen höher als in den bisher verfügbaren natürlichen Quellen. Insbesondere auch aufgrund dieser neuen biotechnologischen Entwicklungen besteht seitens der Industrie ein erhebliches Interesse an Extraktionsverfahren, die es ermöglichen, das natürliche β-Carotin aus der komplexen Naturstoffmatrix in wirtschaftlicher Weise zu extrahieren.

Zur Extraktion des β-Carotins aus festen Naturstoffen sind bereits zahlreiche Verfahren bekannt. Wie bspw. in der US-Patentschrift 2 170 872 beschrieben, wird dazu häufig die Naturstoffmatrix mit Alkalilauge behandelt, um so einen Zellaufschluß und die Verseifung der Glyceride zu erzielen. Anschließend werden die aufgeschlossenen Zellen mit Petrolether, Heptan oder ähnlichen Lösemitteln extrahiert. Der Extrakt wird - von Faserstoffen befreit - einer Wasserdampfdestillation unterzogen; anschließend wird der wäßrige Rückstand mit Pflanzenöl extrahiert, in dem das β-Carotin eine verhältnismäßig hohe Löslichkeit besitzt.

Gemäß einem Verfahren, das in der deutschen Offenlegungsschrift DE 43 42 798 beschrieben ist, können Carotinoide aus Algen direkt aus einer hochkonzentrierten Salzsolekultur mit großen Mengen an Speiseöl extrahiert werden, indem man den Stoffübergang durch eine Kolloidmühle begünstigt. Durch Rückführung des extraktbeladenen Öles kann der Wertstoff angereichert werden, der entweder das β-Carotin-haltige Öl sein kann, oder aber das β-Carotin muß erst noch aus dem Öl durch Kristallisation ausgefällt werden. Erfahrungsgemäß ist jedoch die Kristallisation des β-Carotins aus Öl unter Ausnutzung der temperaturbedingten Löslichkeitsunterschiede nicht unproblematisch, da die Kristallbildung nur sehr langsam erfolgt und das Öl auch bei niedrigen Temperaturen eine noch relativ hohe Restlöslichkeit für β-Carotin besitzt, so daß die Kristallisationsausbeuten nur gering sind.

Die Induktion der β-Carotin-Kristallisation durch die Zugabe von Lösemitteln dagegen führt zu grundsätzlich höheren Ausbeuten, jedoch ist die Zugabe von größeren Lösemittelmengen, wie z.B. in der Patentschrift US 1,988,031 für n-Propanol beschrieben, notwendig, um ein entsprechendes Ausbeuteergebnis zu erhalten. Bei der Extraktion aus Naturstoffen mit organischen Lösemitteln, wie z.B. Petrolether (vgl. die US-Patentschriften 1,967,121 und 1,998,031) stellt sich generell das Problem, daß wegen der geringen Löslichkeit des β-Carotins und insbesondere im Fall hoher β-Carotin-Konzentrationen eine überaus große Lösemittelmenge gewählt werden muß, die aber wiederum die Raum-Zeit-Ausbeute deutlich verschlechtert.

Vor kurzem wurden einige Verfahren beschrieben, bei denen die Extraktion des β-Carotins aus Naturstoffen mit überkritischem Kohlendioxid bei sehr hohen Verfahrensdrücken (US 4,400,398) durchgeführt wird. Trotz der guten Extraktionsergebnisse ist der allgemein gültige Nachteil dieser Gas-Extraktionsverfahren in der aufwendigen technischen Umsetzung des benötigten Hochdruckes zu sehen, die generell kostenintensiver ist als Verfahren, die unter Normaldruck arbeiten.

Im Rahmen der Vereinheitlichung der lebensmittelrechtlichen Gesetzgebung innerhalb der EU wurde im Januar 1995 ein Entwurf einer Richtlinie der Kommission zur Festlegung spezifischer Reinheitskriterien für Farbstoffe, die in Lebensmitteln verwendet werden können, vorgelegt. Zur Extraktion von natürlichen Carotinoiden werden dabei die Lösemittel Aceton, Methylethylketon, Methanol, Ethanol, Propan-2-ol, Hexan, Dichlormethan und Kohlendioxid vorgeschlagen. Mit Ausnahme von Dichlormethan sind diese Lösemittel jedoch aufgrund ihres geringen Lösevermögens gegenüber β-Carotin für eine wirtschaftliche Extraktion aus Naturstoffen, in denen das β-Carotin in hohen Konzentrationen vorkommt, wenig geeignet. Auf der anderen Seite sollte aufgrund ökologischer und verbraucherbezogener Aspekte auf die Verwendung von Dichlormethan in der zukunftsorientierten Lebensmittelindustrie verzichtet werden.

Die Aufgabe der vorliegenden Erfindung war es somit, ein möglichst wirtschaftliches Verfahren zur Gewinnung von CarotinFarbstoffen, insbesondere von β-Carotin, aus Naturstoffen, insbesondere aus festen Naturstoffen mit einem flüssigen Extraktionsmittel zu entwickeln, das die Nachteile der bekannten Verfahren umgeht. Dabei soll das Verfahren nach der eigentlichen Extraktion eine gute Kristallisationsausbeute vor allem an β-Carotin ermöglichen. Insbesondere sollen nur solche Lösemittel verwendet werden, die unter ökologischen und toxikologischen Gesichtspunkten als vorteilhaft eingestuft werden.

Gelöst wurde diese Aufgabe mit einem Verfahren zur Extraktion von Carotinfarbstoffen, bei dem das Ausgangsmaterial mit einem Lösemittelgemisch, das Essigsäureester von C₁-C₄-Alkoholen und 1 bis 25 Gew.-% eines Öls biologischen Ursprungs bezogen auf die Essigsäureester enthält, bei Temperaturen von mindestens 30°C extrahiert wird

Auf den ersten Blick erscheinen Essigsäureester im Vergleich zu einem Öl weniger geeignet für die Extraktion von Carotinfarbstoffen, da sie für derartige Verbindungen ein vergleichsweise geringes Lösevermögen besitzen. Überraschenderweise wurde jedoch gefunden, daß sie oberhalb einer Temperatur von 30°C und insbesondere bei einer Temperatur zwischen 40°C und 125°C hervorragend als Extraktionsmittel für Carotinfarbstoffe geeignet sind.

In einem Gemisch von Essigsäureestern auf der einen Seite und Öl auf der anderen Seite verhält sich die Löslichkeit des Carotins bei Extraktionstemperaturen < 30 °C zunächst auch wie erwartet, nämlich direkt proportional zum Anteil an Öl im Lösemittelgemisch; d.h. die Löslichkeit der Carotine im Gemisch kann sehr einfach graphisch durch Interpolation der Löslichkeit in reinem Ethylacetat oder Butylacetat und Öl ermittelt werden. Überraschenderweise wurde mit dem erfindungsgemäßen Verfahren jedoch festgestellt, daß sich die Löslichkeiten von Carotinfarbstoffen, insbesondere von β-Carotin, in Essigsäureestern bei erhöhten Temperaturen bereits durch den Zusatz geringer Mengen Öl sehr stark erhöhen läßt, was überhaupt nicht zu erwarten war.

Bevorzugt werden im erfindungsgemäßen Verfahren die Essigester von C₂-C₄-Alkoholen eingesetzt. Besonders bevorzugt sind als Essigester die gemäß der - zwischenzeitlich europaweit gültigen - Extraktionslösungsmittel-VO vom 08.11.1991, Anlage 1 allgemein bei der Herstellung von Lebensmitteln einsetzbaren Lösemittel Ethylacetat, Butylacetat und Gemische davon.

Erfindungsgemäß haben sich Öle biologischen Ursprungs für das vorliegende Verfahren als besonders geeignet erwiesen. Mögliche pflanzliche Öle sind Maiskeimöl, Sojaöl, Baumwollsaatöl, Rapsöl oder auch Erdnußöl; es sind aber auch mikrobielle Öle aus bestimmten Pilzen, Hefen oder Bakterien ebenso geeignet wie Fischöle, die für ein Produkt in Lebensmittelqualität eingesetzt werden können. In Ausnahmefällen kann es für das vorliegende Verfahren von Vorteil sein, wenn der natürliche Ölgehalt im Ausgangsmaterial durch entsprechende Entölungsmaßnahmen oder den Zusatz von Öl vor Beginn der Extraktion auf geeignete Werte eingestellt wird.

Durch den Zusatz an Öl von weniger als 5 Gew.-% im Lösemittelgemisch ist bereits eine wesentliche Steigerung der Löslichkeit der Farbstoffe zu erreichen. Erfindungsgemäß wird das Öl deshalb bevorzugt in Anteilen von mindestens 3 Gew.-% und besonders bevorzugt zwischen 5 und 10 Gew.-% im Lösemittelgemisch eingesetzt. Über einem Anteil von 10, vor allem aber 15 Gew.-% Öl kann zwar die Löslichkeit der Carotine im Lösemittelgemisch noch weiter gesteigert werden, jedoch kann der entscheidende Vorteil des Verfahrens, nämlich die hohen Ausbeuten an Carotinfarbstoffen, die nach der Extraktion isoliert werden können, verloren gehen. Bei geringeren Ölkonzentrationen im Gemisch wird zwar die Ausbeute bei der nachfolgenden Kristallisation des Carotins noch gesteigert, jedoch ist zum Erreichen des Extraktionsziels ein höherer Ölanteil im Lösemittel bevorzugt.

Für die Extraktion mit dem Lösemittel-Ölgemisch haben sich Temperaturen von ≥ 50 °C als besonders geeignet erwiesen, da die Verbesserung der Löslichkeit, die durch Ölzugabe erreicht werden kann, bei erhöhten Temperaturen noch stärker ausgeprägt ist; die obere Grenze der Verfahrenstemperatur ist selbstverständlich der Siedepunkt des jeweiligen Lösemittels (Ethylacetat: 77°C, Butylacetat: 123 - 126°C), die verfahrenstechnisch aber im allgemeinen nicht ereicht wird. Beim erfindungsgemäßen Verfahren liegt der besonders geeignete Temperaturbereich für die Extraktion somit zwischen 50 und 70°C. Pro Gew.-Teil festes Ausgangsmaterial werden vorzugsweise 3 bis 5 Gew.-Teile an Lösemittelgemisch zugegeben, worauf dieses Extraktionsgemisch in der Regel für die Dauer von 1 bis 3 Stunden behandelt wird, was am einfachsten durch Rühren geschieht. Nach erfolgter Abtrennung des Lösemittelgemisches bspw. durch Zentrifugation wird der Extraktionsrückstand vorzugsweise mit 0,5 bis 5 Gew.-Teilen Essigsäureester, z.B. Ethylacetat und/oder Butylacetat, nachgewaschen, was auch mehrfach geschehen kann, und die Lösemittelmengen aus dem Nachwaschvorgang mit den vom Öl abgetrennten Lösemittelmengen vereint. Die Gewinnung des reinen Carotinfarbstoffes geschieht nach den bekannten Verfahren, indem bspw. im Vakuum oder durch Anlegen eines Temperaturgradienten die organischen Lösemittel abgezogen werden, wodurch das Carotin als Feststoff anfällt.

Der wesentliche Vorteil des neuen Verfahrens besteht darin, daß einerseits ein dem Öl vergleichbares Lösevermögen für Carotinfarbstoffe erreicht werden kann, wodurch die Raum-Zeit-Ausbeuten deutlich verbessert werden, da insgesamt weniger Lösemittel benötigt wird; andererseits ist es aber durch den nur geringen Anteil des Öls im Lösemittelgemisch möglich, nach der Extraktion und der Entfernung des Lösemittelgemisches eine deutlich höhere Ausbeute an kristallinem Carotinfarbstoff zu erreichen, da die absoluten Verluste durch die Restlöslichkeit des Carotins im geringen Ölanteil nur sehr klein sind. Erfindungsgemäß ist die entscheidende verfahrenstechnische Neuheit für die Extraktion von Carotinfarbstoffen, besonders von β-Carotin, aus Naturstoffen darin zu sehen, daß das Verfahren die Vorteile einer reinen Ölextraktion, nämlich die günstigen Raum-Zeit-Ausbeuten durch die hohe Löslichkeit der Carotinfarbstoffe, insbesondere im Öl, und die Vorteile der reinen Lösemittelextraktion, nämlich die hohen Kristallausbeuten, vereint.

Die nachfolgenden Beispiele sollen die Vorteile des erfindungsgemäßen Verfahrens verdeutlichen.

### Beispiele

Jeweils ein Gew.-Teil eines zuvor entfetteten und getrockneten Fermentationsrückstandes eines β-Carotin-produzierenden sproßbildenden Pilzes (β-Carotingehalt 3,0 Gew.-%) wurde mit jeweils 4 Gew.-Teilen der in Tab. 1 genannten Lösemittel bzw. Lösemittelgemische über einen Zeitraum von 2 h bei den angegebenen Verfahrenstemperaturen kräftig gerührt. Anschließend wurde die Biomasse über eine Tuchzentrifuge abgetrennt und mit 1 Gew.-Teil des auf Extraktionstemperatur gebrachten Lösemittels bzw. Lösemittelgemischs nachgewaschen. Nachdem das Lösemittel(-gemisch) aus den vereinten Carotin-haltigen Extraktionslösungen im Vakuum entfernt worden war, wurde die verbliebene Konzentration an β-Carotin nach dem Trocknen im Rückstand bestimmt und die Extraktionsausbeute ermittelt. Als Ölkomponente wurde ein Maiskeimöl in Speiseölqualität eingesetzt.

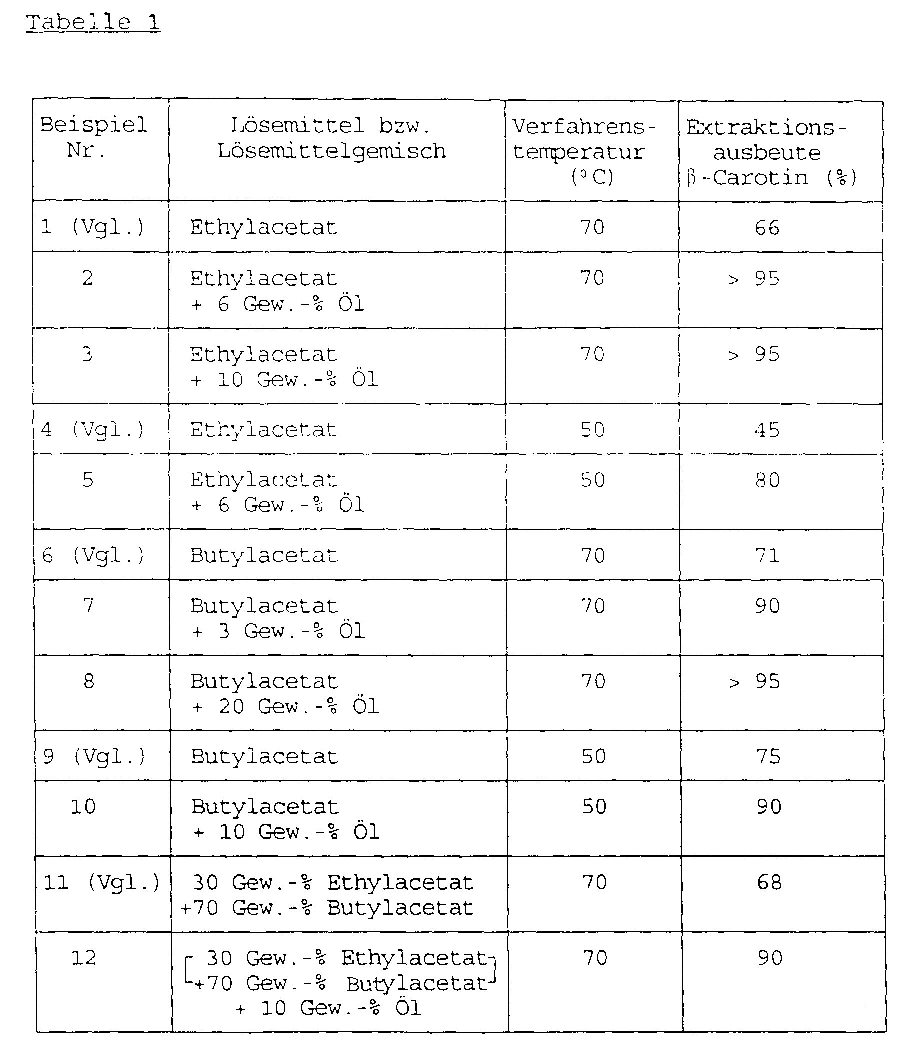

## Patentansprüche

1. Verfahren zur Extraktion von Carotinfarbstoffen, insbesondere von β-Carotin, aus Naturstoffen mit einem flüssigen organischen Extraktionsmittel,
**dadurch gekennzeichnet,**
daß das Ausgangsmaterial mit einem Lösemittelgemisch, das Essigsäureester von C1-C4-Alkoholen und 1 bis 25 Gew.-% eines Öls biologischen Ursprungs bezogen auf die Essigsäureester enthält, bei Temperaturen von mindestens 30°C extrahiert wird.

2. Verfahren nach Anspruch 1,
**dadurch gekennzeichnet,**
daß die Essigsäureester aus Ethylacetat, Butylacetat und Gemischen davon ausgewählt werden.

3. Verfahren nach einem der Ansprüche 1 bis 2,
**dadurch gekennzeichnet,**
daß das Öl in Anteilen zwischen 5 und 10 Gew.-% im Lösemittelgemisch eingesetzt wird.

4. Verfahren nach einem der Ansprüche 1 bis 3,
**dadurch gekennzeichnet,**
daß die Extraktion bei Temperaturen zwischen 50 und 70°C durchgeführt wird.

5. Verfahren nach einem der Ansprüche 1 bis 4,
**dadurch gekennzeichnet,**
daß pro Gew.-Teil Ausgangsmaterial 3 bis 5 Gew.-Teile an Lösemittelgemisch zugegeben werden.

6. Verfahren nach einem der Ansprüche 1 bis 5,
**dadurch gekennzeichnet,**
daß das Extraktionsgemisch 1 bis 3 Stunden behandelt wird.

7. Verfahren nach einem der Ansprüche 1 bis 6,
**dadurch gekennzeichnet,**
daß der Extraktionsrückstand mit 0,5 bis 5 Gew.-Teilen Essigsäureester nachgewaschen wird.

## Claims

1. Process for the extraction of carotene dyes in particular of β-carotene from natural products using a liquid organic extracting agent,
**wherein**
the starting material is extracted at temperatres of at least 30°C with a solvent mixture which contains acetic acid ester of C₁-C₄ alcohols and 1 to 25 % by weight relative to the acetic acid esters of an oil from a biological source.

2. Process as claimed in claim 1,
**wherein**
the acetic acid esters are selected from ethyl acetate, butyl acetate and mixtures thereof.

3. Process as claimed in one of the claims 1 to 2,
**wherein**
the oil is used in portions between 5 and 10 % by weight in the solvent mixture.

4. Process as claimed in one of the claims 1 to 3,
**wherein**
the extraction is carried out at temperatures between 50 and 70°C.

5. Process as claimed in one of the claims 1 to 4,
**wherein**
3 to 5 parts by weight solvent mixture are added per part by weight of starting material.

6. Process as claimed in one of the claims 1 to 5,
**wherein**
the extraction mixture is treated for 1 to 3 hours.

7. Process as claimed in one of the claims 1 to 6,
**wherein**
the extraction residue is rewashed with 0.5 to 5 parts by weight acetic acid ester.

## Revendications

1. Procédé d'extraction de colorants carotènes, en particulier de β-carotène, à partir de produits naturels, avec un agent d'extraction organique liquide, caractérisé en ce que l'on extrait le produit de départ avec un mélange de solvants contenant des esters d'acide acétique et d'alcools en C₁-C₄ et 1 à 25 % en masse par rapport aux esters d'acide acétique d'une huile d'origine biologique, à des températures d'au moins 30°C.

2. Procédé selon la revendication 1, caractérisé en ce que les esters d'acide acétique sont choisis parmi l'acétate d'éthyle, l'acétate de butyle et leurs mélanges.

3. Procédé selon l'une des revendications 1 à 2, caractérisé en ce que l'on utilise l'huile en des proportions comprises entre 5 et 10 % en masse dans le mélange de solvants.

4. Procédé selon l'une des revendications 1 à 3, caractérisé en ce que l'extraction s'effectue à des températures comprises entre 50 et 70°C.

5. Procédé selon l'une des revendications 1 à 4, caractérisé en ce que l'on ajoute 3 à 5 parties en masse de mélange de solvants par partie en masse de produit de départ.

6. Procédé selon l'une des revendications 1 à 5, caractérisé en ce que l'on traite le mélange d'extraction pendant 1 à 3 heures.

7. Procédé selon l'une des revendications 1 à 6, caractérisé en ce que l'on lave ensuite le résidu d'extraction avec 0,5 à 5 parties en masse d'ester d'acide acétique.
